# FASCICULE DE BREVET EUROPEEN

(11) **EP 0 685 473 B1**
(45) Date de publication et mention de la délivrance du brevet: **14.05.1997**
(21) Numéro de dépôt: 95400577.3
(22) Date de dépôt: 15.03.1995
(51) Int. Cl.: C07D 307/79, C07D 311/58, C07D 313/08, C07D 313/04, C07C 319/20, A61K 7/48

(54) **Composés benzohétérocycliques, en tant qu'antioxydants**
Benzoheterocyclische Verbindungen als Antioxidantien
Benzoheterocyclic compounds, as antioxidants

(30) Priorité: 31.05.1994 FR 9406616
(43) Date de publication de la demande: 06.12.1995
(73) Titulaire: L'OREAL, 75008 Paris (FR)
(72) Inventeur: Solladie, Guy, F-67000 Strasbourg (FR); Boeffel, Dominique, F-67300 Schiltigheim (FR); Maignan, Jean, F-93290 Tremblay-en-France (FR)
(74) Mandataire: Dodin, Catherine

(56) Documents cités:
- EP-A- 0 150 291
- EP-A- 0 413 668
- WO-A-80/02098
- GB-A- 2 188 634
- GB-A- 2 221 680

## Description

La présente invention a trait à de nouveaux composés de la famille des benzohétérocycles ainsi qu'à leurs procédés de préparation et à leur utilisation entre autre dans le domaine cosmétique.

Il est connu, par la demande GB-A-2221680 des dérivés de 5-hydroxy-2,3-dihydrobenzofuranes.

La présente invention a pour but de proposer des procédés nouveaux, simples et rapides, permettant la préparation de composés nouveaux de la famille des benzohétérocycles, avec un bon rendement.
La présente invention a également pour but de proposer des composés nouveaux, pouvant être utilisés entre autre dans des compositions cosmétiques.

La présente invention a donc pour premier objet les composés chimiques de formule (I) 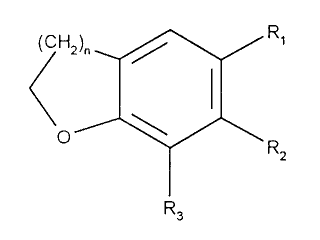 dans laquelle
. n est égal à 1, 2 ou 3,
. R1 est un groupement -SR4 ou -OR4, R4 étant un radical alkyle ayant 1 à 6 atomes de carbone,
. R2 est un groupement -OH, un radical alkyle ayant 1 à 6 atomes de carbone ou un radical alcoxy ayant 1 à 6 atomes de carbone, et
. R3 est un atome d'hydrogène ou un radical alkyle ayant 1 à 6 atomes de carbone,
R1 et R2 ne pouvant être simultanément des groupements -OCH3 lorsque R3 est un atome d'hydrogène et n=2 ou 3.
Par radical alkyle, on entend dans la présente description, tout radical hydrocarboné saturé.

Ces composés chimiques intermédiaires sont de formule (II) 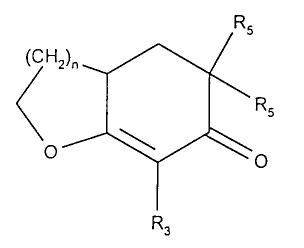 dans laquelle n est égal à 1, 2 ou 3, R5 est, de manière indépendante, un groupement -SR4 ou -OR4, R4 étant un radical alkyle ayant 1 à 6 atomes de carbone, et R3 est un atome d'hydrogène ou un radical alkyle ayant 1 à 6 atomes de carbone.

La réaction-clé du procédé de synthèse des composés de formule (I) selon l'invention est l'anhélation 3C+3C de Michael Claisen d'une α-méthylènelactone avec l'anion d'un α-cétothioacétal.
On obtient ainsi un mélange céto-énolique non purifiable, qui peut être directement transformé en produit α-alkylthio-β-hydroxyaromatique, ou bien en α-alkylthio-β-alcoxyaromatique, ou encore en α-alkylthio-β-alkylaromatique, par déshydratation suivie de désulfuration dans le benzène à reflux en présence d'une quantité catalytique d'acide p-toluène sulfonique.
Ledit mélange céto-énolique peut aussi être partiellement déshydraté en dialkylthioacétal à groupement dihydrofuranne ou pyranne, dans l'acide acétique à chaud. Le dialkylthioacétal peut alors être transformé en dialcoxyacétal par l'acétate de mercure dans un mélange méthanol-chloroforme.
L'hydrolyse acide de ce dialcoxyacétal peut conduire aux α-alcoxy-β-alcoxyaromatiques alors que l'addition de méthyl-lithium suivie d'une hydrolyse acide permet l'accès aux α-alcoxy-β-alkylaromatiques.

On a de plus constaté que les composés de formule (I) selon l'invention présentent certaines propriétés antioxydantes et peuvent être utilisés comme agent antioxydant, par exemple dans des compositions cosmétiques telles que des lotions ou des crèmes.
L'invention a donc également pour objet l'utilisation des composés de formule (I) comme agent antioxydant, ainsi qu'une composition cosmétique ou pharmaceutique le comprenant.
En particulier, les composés de formule (I) substitués par deux fonctions, l'une phénol (R2= -OH) et l'autre thioéther (R1= -SR4), peuvent agir à deux niveaux de la peroxydation des lipides, la fonction phénol inhibant l'étape de propagation, et la fonction thioéther réduisant les peroxydes et/ou les hydroperoxydes formés.

Ces composés peuvent donc être utilisés aussi bien en tant que conservateur dans des compositions cosmétiques ou pharmaceutiques, qu'en tant qu'agent susceptible de prévenir les effets du vieillissement et/ou du photovieillissement in vivo, par application topique.

L'invention est illustrée plus en détail dans les exemples suivants qui décrivent les procédés de préparation de plusieurs composés selon l'invention.
Dans ces exemples, les spectres RMN du proton et du carbone 13 sont effectués dans le trichlorodeutérométhane, respectivement à 200 et 50 MHz.

### Exemple 1 : Synthèse de trois composés α-alcoxy-β-alcoxyaromatiques de formule

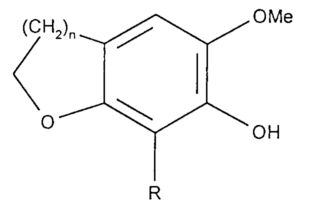

La synthèse de ces trois composés est effectuée en plusieurs étapes, consistant
. à préparer certains produits de départ (exemples a et b),
. à préparer à partir de ces produits de départ, les premiers intermédiaires de formule (II) avec R5= -SR4 (exemple c), puis
. à préparer à partir de ces premiers intermédiaires, les seconds intermédiaires de formule (II) avec R5= -OR4 (exemple d), et
. à préparer à partir de ces seconds intermédiaires, les composés de formule (I) selon l'invention (exemple e).

Les produits de départ utilisés sont des α-méthylène lactones et des α-cétothio-acétals.
Parmi les α-méthylène lactones, on utilise dans les exemples suivants :
. l'α-méthylène-γ-butyrolactone, que l'on peut synthétiser selon la méthode de UENO et al, Tetrahedron Lett., 39, 3753-58 (1978) en une seule étape à partir de l'α-acétyl-γ-butyrolactone commerciale.
. l'α-méthylène-δ-valérolactone, synthétisée selon la méthode de G.M. KSANDER et al, J. Org. Chem., 42, 1180-85 (1977) en deux étapes au départ de la δ-valérolactone commerciale.
. l'α-méthylène-ε-caprolactone

Pour ce composé, une nouvelle synthèse plus courte et plus performante que celle de l'état de la technique (Mori et al, J. Org. Chem., 48, 4058-67 (1983)) qui ne donnait qu'un rendement de 4% et nécessitait une étape de carbonylation sur palladium, a été mise au point en s'inspirant de la méthodologie développée par I. I. PATERSON et al, Chem. Ber., 11, 993-94 (1979) pour les α-méthylène-cétones et l'α-méthylène-δ-valérolactone.
Cette synthèse est décrite dans l'exemple a.

Parmi les α-cétothioacétals employés pour la synthèse des composés selon l'invention, on utilise dans les exemples suivants:
. la 1,1-bis-(méthylthio)-2-propanone pour laquelle une nouvelle synthèse a été mise au point.
   On connaît une seule synthèse de ce produit, décrite dans la littérature par BOHME et al, Arch. Pharmaz., 9, 282 (1944) à partir de méthylmercaptan, de 1,1-dichloroacétone et d'éthylate de sodium, avec un rendement de 60%.
   Pour éviter l'utilisation du méthylmercaptan, une approche par décarboxylation d'un diméthylthiocétoester a été développée.
   Cette synthèse est décrite dans l'exemple b.
. la 1,1-bis-(méthylthio)-2-butanone, synthétisée en une étape par addition de l'anion du bis(méthylthio)méthane commercial au propionate d'éthyle, avec un rendement de 98%, sachant qu'il faut deux équivalents d'anion car le produit d'arrivée a un proton plus acide que le produit de départ.

### Exemple a : synthèse de l'α-méthylène-ε-caprolactone

Cette synthèse est effectuée par réaction de l'énol silylé de l'ε-caprolactone avec du chlorométhylphénylsulfure en présence d'une quantité catalytique de chlorure de zinc pour conduire au composé sulfuré, qui est oxydé en sulfoxyde par le métapériodate de sodium. La pyrolyse du sulfoxyde dans le toluène à reflux conduit à l'α-méthylène-ε-caprolactone.

Dans un bicol flambé sous argon, de la diisopropylamine (2,82 ml; 20 mmol) est mise en solution dans 20 ml de tétrahydrofuranne (THF) anhydre.
Après refroidissement à -78°C, du n-butyl-lithium (14,65 ml d'une solution 1,37M dans l'hexane; 20 mmol) est ajouté au goutte à goutte rapide. Après 30 minutes d'agitation à -78°C, l'ε-caprolactone fraîchement distillée (2,21 ml; 20 mmol) est ajoutée goutte à goutte. La solution résultante est agitée pendant 30 min à -78°C puis le chlorure de triméthylsilyle (6,54 ml; 50 mmol) est additionné goutte à goutte. Le bain froid est retiré et le milieu est encore agité pendant 30 minutes.
La solution est alors filtrée rapidement sur un fritté de porosité 4 et le solvant est évaporé.
On dissout le résidu dans de l'éther anhydre (20 ml) puis on le filtre sur fritté pour enlever les sels. L'opération est réitérée jusqu'à ce qu'il n'y ait plus de sels qui précipitent (3 fois en général), puis le solvant est évaporé sous pression réduite.
Le composé silylé s'hydrolyse très vite. Il est donc préférable de le remettre directement en réaction.
Un échantillon est toutefois distillé (distillateur à boules, température d'ébullition: environ 60°C, pression: environ 0,5 mbar) de manière à analyser le produit obtenu.

On obtient les résultats suivants:
. rendement après distillation : 65% (2,51 g; 13,5 mmol)
. masse moléculaire : M= 186
. le spectre RMN du proton donne les résultats suivants:

| proton | δ (ppm) |
|---|---|
| Si(CH₃)₃ | 0,09 (s; 9H) |
| H₄ | 1,51 (m; 2H) |
| H₅ | 1,70 (m; 2H) |
| H₃ | 1,89 (m; 2H |
| H₆ | 3,86 (t; 2H; J=5 Hz) |
| H₂ | 3,98 (t; 2H; J=6 Hz) |

Les données obtenues permettent donc d'identifier le composé comme étant l'énol silylé de l'ε-caprolactone, de formule

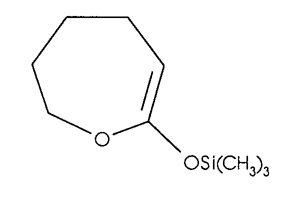

Dans un bicol flambé sous argon contenant du bromure de zinc anhydre (0,021 g; 0,092 mmol), sont ajoutés successivement du dichlorométhane anhydre (20 ml), I'énol silylé ci-dessus (1,715 g; 9,22 mmol) et du chlorométhyl phényl sulfure (1,85 ml; 13,8 mmol).
La solution est agitée pendant 36 heures à température ambiante, puis le solvant est évaporé sous pression réduite. Le résidu est purifié par chromatographie éclair sur colonne de silice (éluant : gradient par 10% dichlorométhane/hexane 60/40 à dichlorométhane pur) pour donner après concentration une huile jaune visqueuse.
Le rendement est de 52% (1,14 g; 4,83 mmol) si l'on utilise l'énol silylé distillé, et de 44% sur les deux étapes si l'énol silylé n'est pas distillé.

Les spectres RMN du proton et du carbone 13 donnent les résultats suivants:

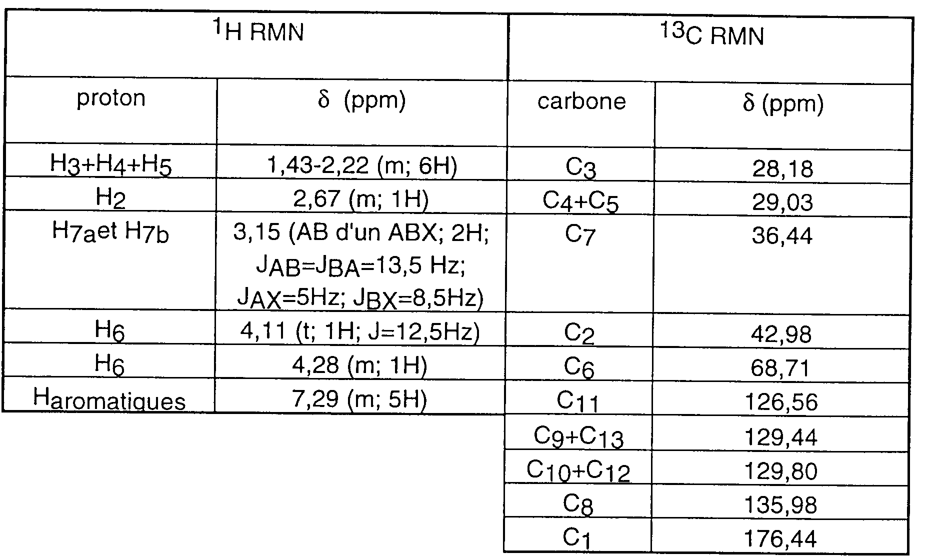

Les données obtenues permettent donc d'identifier ce composé comme étant l'α-phénylthiométhyl-ε-caprolactone de formule

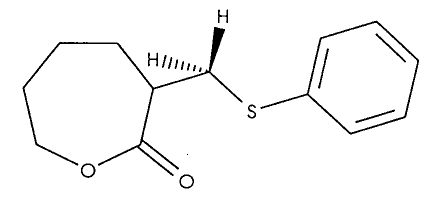

On dissout le composé ci-dessus (11 g; 46,61 mmol) dans un mélange méthanolbenzène 13/1 (350 ml). La solution résultante est refroidie à 0°C par un bain de glace. On dissout du métapériodate de sodium (12,95 g; 60,6 mmol) dans de l'eau distillée (140 ml), et on l'ajoute, au goutte à goutte rapide. Le bain de glace est enlevé et la solution est agitée à température ambiante pendant 24 heures, puis extraite au dichlorométhane (5 x 100 ml). Les phases organiques sont rassemblées, lavées à l'eau, séchées sur sulfate de sodium et évaporées sous pression réduite.
Le résidu est purifié par chromatographie éclair sur colonne de silice avec un mélange dichlorométhane/méthanol 99/1 pour conduire à une huile visqueuse jaune pâle. Le rendement est donc de 97,5% (11,45 g; 45,44 mmol)

Les spectres RMN du proton et du carbone 13 donnent les résultats suivants:

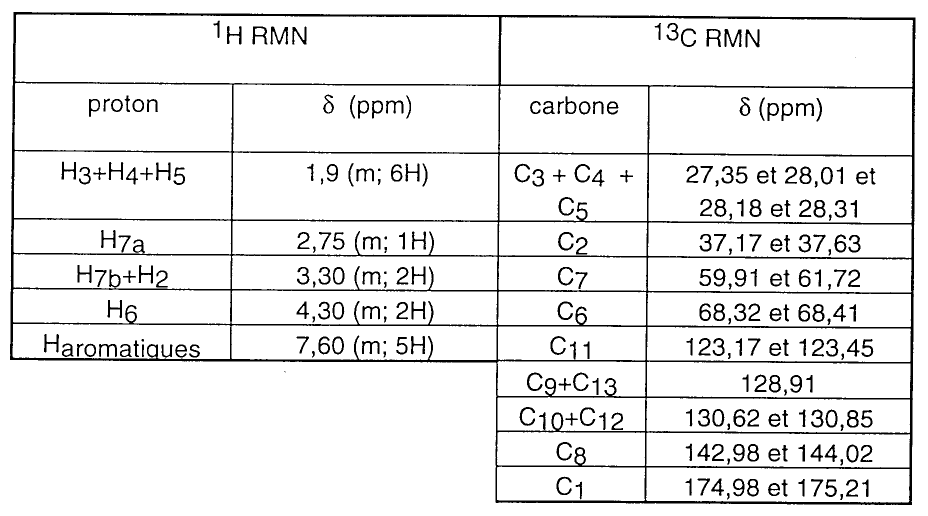

Le spectre IR du composé, effectué dans CCl₄ donne les résultats suivants:

| bandes | cm⁻¹ |
|---|---|
| C-H | 3020-2840 |
| C=O | 1720 |

La microanalyse donne les résultats suivants:

| | calculé | trouvé |
|---|---|---|
| % C | 61,88 | 61,91 |
| % H | 6,39 | 6,43 |

Les données ainsi obtenues permettent donc d'identifier ce composé comme étant l'α-phénylsulfinylméthyl-ε-caprolactone de formule 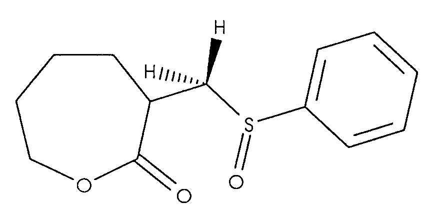 Une solution du composé ci-dessus (0,95 g; 3,77 mmol) dans le toluène (30 ml) est chauffée à reflux pendant 48 heures. Le solvant est évaporé sous pression réduite et le résidu est distillé au distillateur à boules.
On obtient un rendement de 60% (0,286 g; 2,27 mmol)

Les spectres RMN du proton et du carbone 13 donnent les résultats suivants:

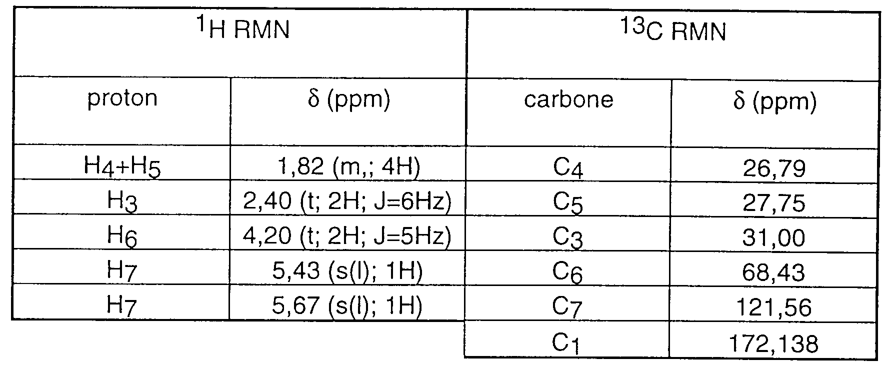

Les données obtenues permettent d'identifier le composé comme étant l'α-méthylène-ε-caprolactone de formule

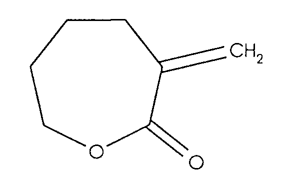

### Exemple b : synthèse de la 1,1-bis(méthylthio)-2-propanone

Dans une première étape, on prépare la 2,2-bis(méthylthio)-acétoacétate de t-butyle de la manière suivante.
Dans un bicol muni d'un condenseur et flambé sous argon, on prépare une solution d'acétoacétate de t-butyle (13,3g; 84 mmol) dans le diméthylformamide (300 ml). A cette solution sont successivement ajoutés du diazo-bicyclo-undécane (DBU) (25,13 ml; 168 mmol) et du thiotosylate de méthyle (34g; 168 mmol).
Le mélange est chauffé à 80°C pendant 30 minutes, refroidi puis versé sur de l'eau glacée (800 ml). Après extraction avec un mélange éther-hexane 50/50 (3 x 200 ml), les phases organiques sont rassemblées, lavées avec une solution aqueuse d'HCl à 10% puis à l'eau distillée, séchées et évaporées sous pression réduite pour conduire à des cristaux jaunes. Le rendement est de 95% (19,9 g).

Les spectres RMN du proton et du carbone 13 donnent les résultats suivants:

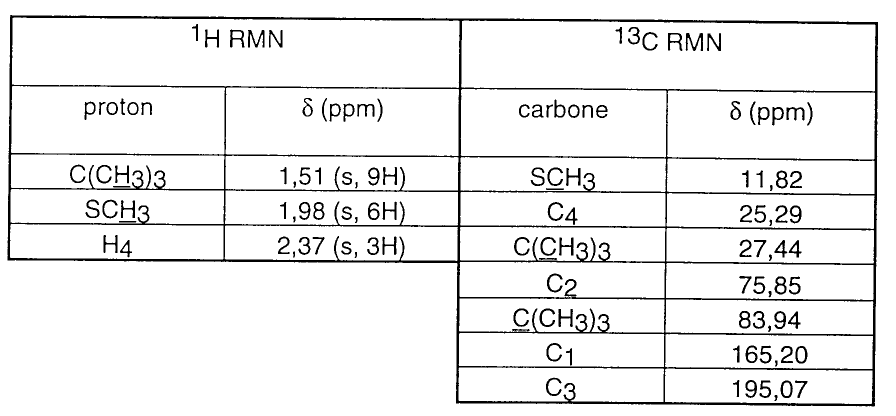

Le spectre IR du composé, effectué dans CCl₄ donne les résultats suivants:

| bandes | cm⁻¹ |
|---|---|
| C-H | 3020-2840 |
| C=O | 1720 |

La microanalyse donne les résultats suivants:

| | calculé | trouvé |
|---|---|---|
| % C | 47,97 | 48,13 |
| % H | 7,25 | 7,39 |

Les données obtenues permettent d'identifier le composé comme étant le 2,2-bis(méthylthio)-acétoacétate de t-butyle, de formule 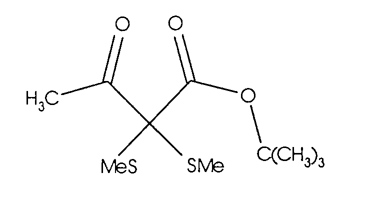 Une solution de DMSO, de 2,2-bis(méthylthio)-acétoacétate de t-butyle (19,9 g; 77 mmol) et d'eau distillée (1,43 ml; 77 mmol) est chauffée à reflux à 160°C pendant 4 heures. Après extraction au dichlorométhane (3 x 100 ml) du mélange refroidi, la phase organique est lavée à l'eau distillée, séchée sur sulfate de sodium et évaporée sous pression réduite.
Le composé brut obtenu est distillé sous vide au distillateur à boules.
On obtient un rendement de 85% (9,8 g; 65,4 mmol).

Les spectres RMN du proton et du carbone 13 donnent les résultats suivants:

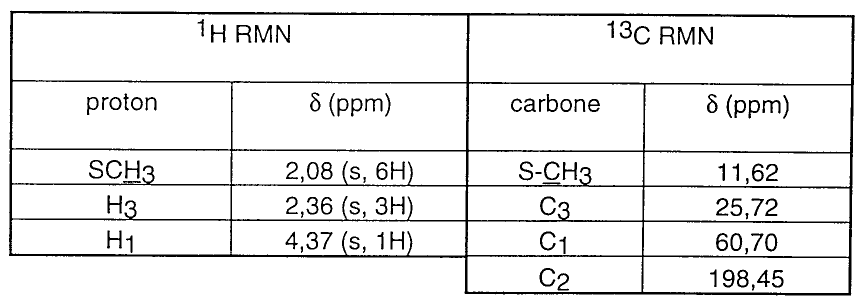

Le spectre IR du composé, effectué dans CCl₄ donne les résultats suivants:

| bandes | cm⁻¹ |
|---|---|
| C-H | 3020-2820 |
| C=O | 1720 |

La microanalyse donne les résultats suivants:

| | calculé | trouvé |
|---|---|---|
| % C | 39,99 | 39,62 |
| % H | 6,71 | 6,71 |

Ces données permettent d'identifier le composé comme étant la 1,1-bis(méthylthio)-2-propanone, de formule :

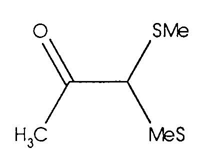

### Exemple c : préparation des premiers intermédiaires de formule

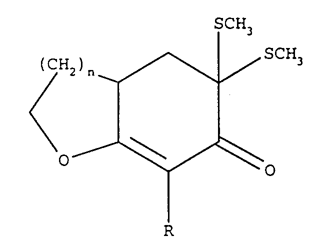

Dans un bicol flambé sous argon et muni d'un condenseur, une suspension de NaH dégraissé dans le dichlorométhane (1 ml/mmol de NaH) est refroidie à 0°C à l'aide d'un bain de glace. On dissout l'α-cétothioacétal correspondant (1 mole pour 2 moles de NaH) dans le dichlorométhane (1 ml/mmol thioacétal), et on l'ajoute, au goutte à goutte à la canule, à la suspension de NaH.
Quand le dégagement d'hydrogène est terminé, l'α-méthylène-lactone correspondante (1 mole pour 2 moles de NaH) dissoute dans le dichlorométhane (1 ml/mmol lactone) est ajoutée sur l'anion du thioacétal goutte à goutte. Après la fin de l'addition, le bain de glace est remplacé par un bain d'huile et le milieu est chauffé à reflux pendant 16 heures.
Après refroidissement à température ambiante, la suspension est versée sur une solution aqueuse d'HCl 10% (10 ml/mmol de thioacétal). La phase organique est décantée et la phase aqueuse est extraite au dichlorométhane.
Les phases organiques rassemblées sont lavées par une solution aqueuse saturée en NaCl, séchées sur sulfate de magnésium puis concentrées.
On dissout le résidu dans de l'acide acétique glacial (5 ml/mmol de produit), puis on le chauffe à 60°C pendant 1 heure. L'acide acétique est ensuite évaporé sous pression réduite et le résidu est chromatographié sur colonne de silice avec l'éluant adéquat.

On synthétise de cette manière les quatre composés suivants:
. le 2,3,9,4-tetrahydro-5,5-bis(méthylthio)-6-one-benzofuranne (n=1, R=H)
Pour la chromatographie, on utilise comme éluant le CH₂Cl₂/MeOH 99/1.
Un échantillon analytique est recristallisé dans un mélange CH₂Cl₂/hexane.
On obtient, avec un rendement de 50% environ, des cristaux blancs dont le point de fusion est de 111-111,5°C.
Le spectre RMN du proton donne les résultats suivants:

| proton | δ (ppm) |
|---|---|
| H_{3b} | 1,83 (ddd; 1H; J_{3b-a}=12Hz; J_{3b-2a}=0; J_{3b-2b}=11,5Hz; J_{3b-9}=7,5Hz) |
| SCH₃ | 2,07 (s; 3H) |
| SCH₃ | 2,13 (s; 3H) |
| H_{4b} | 2,28 (B d'un ABX; 1H; J_{4b-4a}=13Hz; J_{4b-X}=11,5Hz) |
| H₃ₐ | 2,35 (dddd; 1H; J_{3a-3b}=12Hz; J₃ₐ₋₂ₐ=11,5Hz; J_{3a-2b}=5Hz; J₃ₐ₋₉=11,5Hz) |
| H₄ₐ | 2,60 (A d'un ABX; 1H; J_{4a-4b}=13Hz; J_{4a-X}=5Hz) |
| H₉ | 3,34 (dtd(d); 1H; J₉₋₄ₐ=11,5Hz; J_{9-4b}=5Hz; J₉₋₃ₐ=11,5Hz; J_{9-3b}=7,5Hz; J₉₋₈=2Hz) |
| H_{2b} | 4,30 (ddd; 1H; J_{2b-2a}=8,5Hz; J_{2b-3a}=5Hz; J_{2b-3b}=11,5Hz) |
| H₂ₐ | 4,56 (t; 1H; J_{2a-2b}=J₂ₐ₋₃ₐ=8,5Hz; J_{2a-3b}=0) |
| H₇ | 5,43 (s(I); 1H) |

Le spectre RMN du carbone 13 donne les résultats suivants:

| carbone | δ(ppm) |
|---|---|
| SCH₃ | 11,05 |
| SCH₃ | 11,85 |
| C₃ | 29,90 |
| C₉ | 37,47 |
| C₄ | 40,40 |
| C5 | 62,39 |
| C₂ | 73,18 |
| C₇ | 97,09 |
| C₈ | 180,40 |
| C₆ | 191,75 |

Le spectre IR du composé, effectué dans CCl₄ donne les résultats suivants:

| bandes | cm⁻¹ |
|---|---|
| C-H | 2980-2820 |
| C=O | 1635 |

La microanalyse donne les résultats suivants:

| | calculé | trouvé |
|---|---|---|
| % C | 52,14 | 52,08 |
| % H | 6,13 | 5,91 |

Ces analyses correspondent à la structure attendue.
. le 2,3,10,5-tetrahydro-6,6-bis(méthylthio)-7-one-benzopyranne (n=2, R=H)
Pour la chromatographie, on utilise comme éluant le CH₂Cl₂/MeOH 99/1.
On obtient, avec un rendement de 50% environ, des cristaux blancs dont le point de fusion est de 102-104°C.
Les spectres RMN du proton et du carbone 13 donnent les résultats suivants: 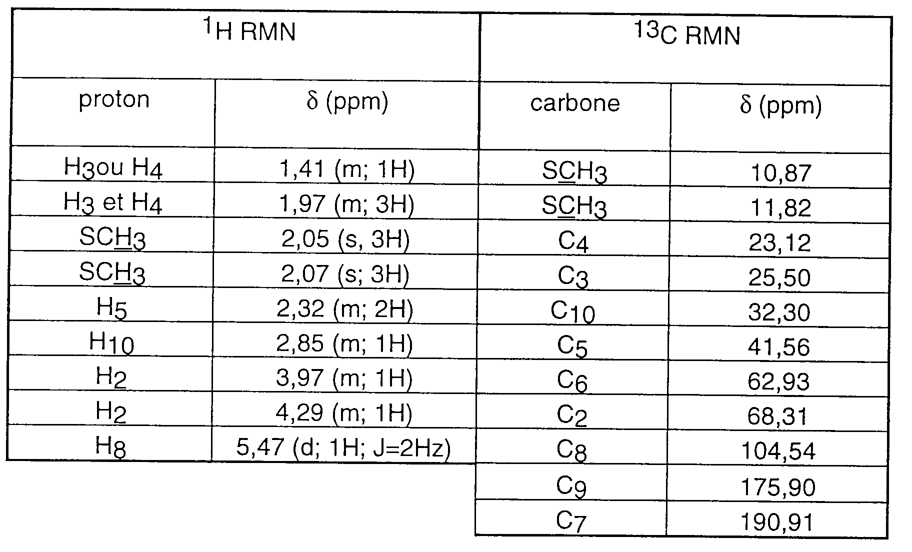
Le spectre IR du composé, effectué dans CCl₄ donne les résultats suivants:

| bandes | cm⁻¹ |
|---|---|
| C-H | 2980-2840 |
| C=O | 1650 |
| C=C | 1615 |

La microanalyse donne les résultats suivants:

| | calculé | trouvé |
|---|---|---|
| % C | 54,07 | 53,85 |
| % H | 6,60 | 6,42 |

Ces analyses correspondent à la structure attendue.
. le 2,3,9,4-tetrahydro-5,5-bis(méthylthio)-6-one-7-méthyl-benzofuranne (n=1, R=CH3)
Pour la chromatographie, on utilise comme éluant CH₂Cl₂. On obtient, avec un rendement de 50%, des cristaux blancs dont le point de fusion est de 98-99°C.
Les spectres RMN du proton et du carbone 13 sont les suivants: 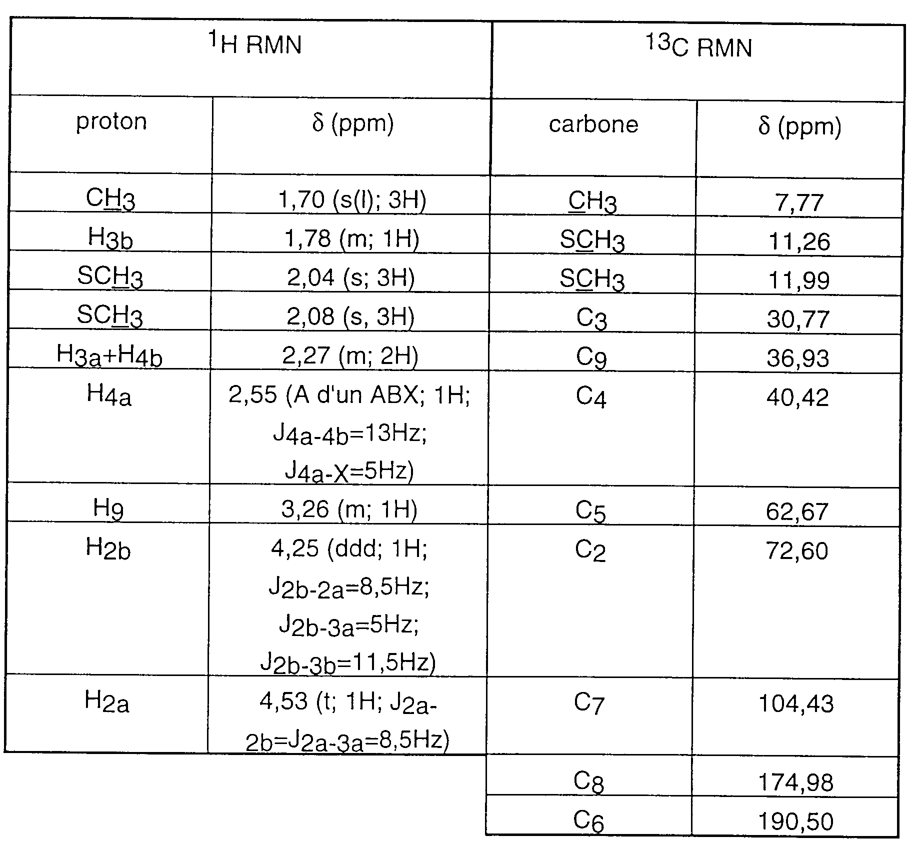
Le spectre IR du composé, effectué dans CCl₄ donne les résultats suivants:

| bandes | cm⁻¹ |
|---|---|
| C-H | 3000-2860 |
| C=O | 1675 |
| C=C | 1640 |

La microanalyse donne les résultats suivants:

| | calculé | trouvé |
|---|---|---|
| % C | 54,07 | 53,88 |
| % H | 6,60 | 6,57 |

Ces analyses correspondent à la structure attendue.
. le 2,3,10,5-tetrahydro-6,6-bis(méthylthio)-7-one-8-méthyl-benzopyranne (n=2, R=CH3)
Pour la chromatographie, on utilise comme éluant un gradient de CH₂Cl₂ à CH₂Cl₂ /MeOH 98/2, par 1%. On obtient, avec un rendement de 58,5% environ, des cristaux blancs dont le point de fusion est de 106-107°C.
Les spectres RMN sont les suivants : 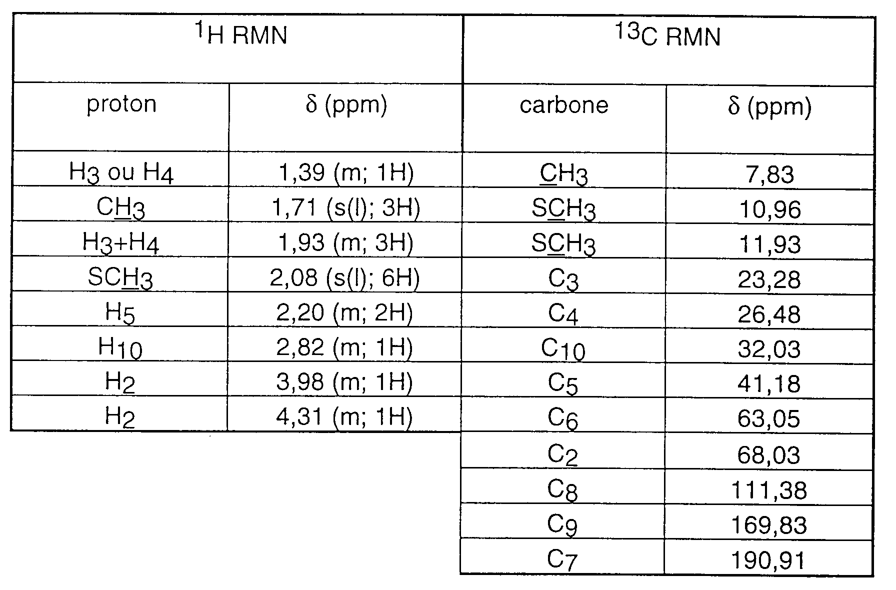
Le spectre IR du composé, effectué dans CCl₄ donne les résultats suivants:

| bandes | cm⁻¹ |
|---|---|
| C-H | 3000-2860 |
| C=O | 1700 |
| C=C | 1650 |

La microanalyse donne les résultats suivants:

| | calculé | trouvé |
|---|---|---|
| % C | 55,78 | 55,95 |
| % H | 7,02 | 6,99 |

### Exemple d : synthèse des seconds intermédiaires de formule

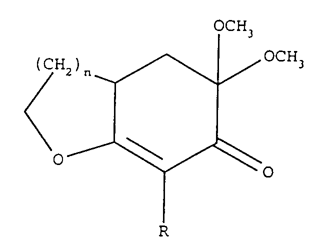

Dans un bicol flambé sous argon, on dissout le dithioacétal cyclique préparé à l'exemple c, dans du chloroforme anhydre (5 ml/mmol). Une solution d'acétate de mercure (2,5 parties pour une partie de dithioacétal) dans le méthanol (2 ml/mmol) est ensuite additionnée au goutte à goutte rapide.
La solution résultante est agitée pendant 16 heures à température ambiante. On observe la formation de sels de mercure insolubles et la solution prend une coloration rose plus ou moins intense.
On évapore les solvants, on dissout le résidu dans du dichlorométhane (5 ml/mmol) et on filtre la suspension sur célite pour enlever les sels de mercure.
Le filtrat limpide est lavé avec une solution d'hydrogénosulfite de sodium à 10% puis avec une solution saturée en NaCl. La phase organique est séchée sur sulfate de sodium et concentrée pour conduire à un solide jaune-brun qui est chromatographié sur colonne de silice avec l'éluant adéquat.

On synthétise de cette manière les trois composés suivants:
. le 2,3,9,4-tetrahydro-5,5-bis(méthoxy)-6-one-benzofuranne (n=1, R=H)
Pour la chromatographie, on utilise comme éluant le CH₂Cl₂/MeOH 98/2. Un échantillon analytique est recristallisé dans CH₂Cl₂/MeOH 98:2. On obtient, avec un rendement de 72%, des cristaux rose pâle.
Les spectres RMN du carbone 13 et du proton donnent les résultats suivants: 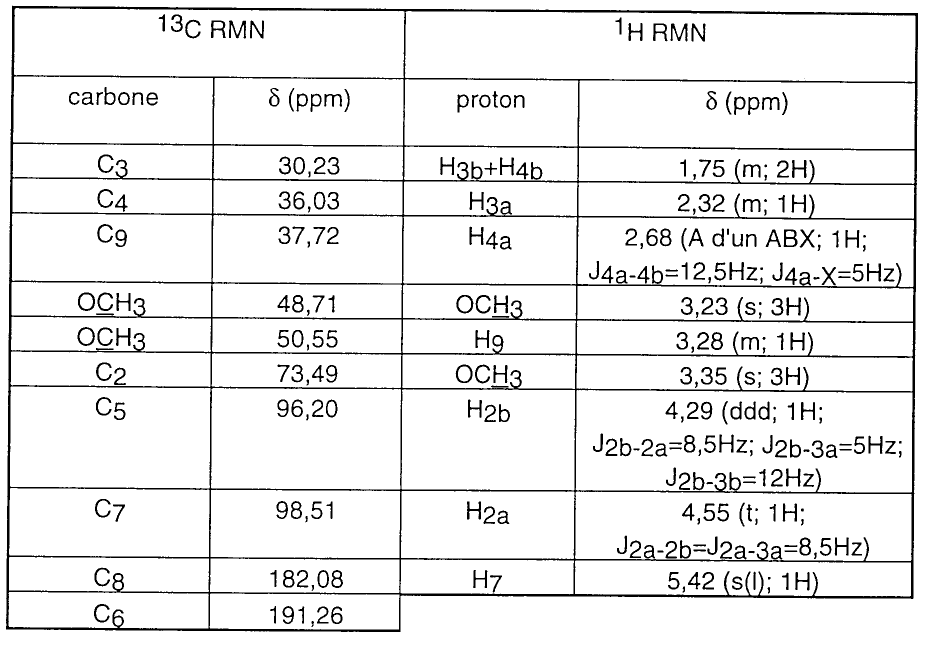
Ces analyses correspondent à la structure attendue.
. le 2,3,9,4-tetrahydro-5,5-bis(méthoxy)-6-one-7-méthyl-benzofuranne (n=1, R=CH3)
L'éluant pour la chromatographie est AcOEt/hexane 1/1. Un échantillon analytique est recristallisé dans AcOEt/hexane 1/1. On obtient des cristaux rose pâle, blancs après recristallisation, dont le point de fusion est de 97-98°C, avec un rendement de 76%.
Les spectres RMN sont les suivants : 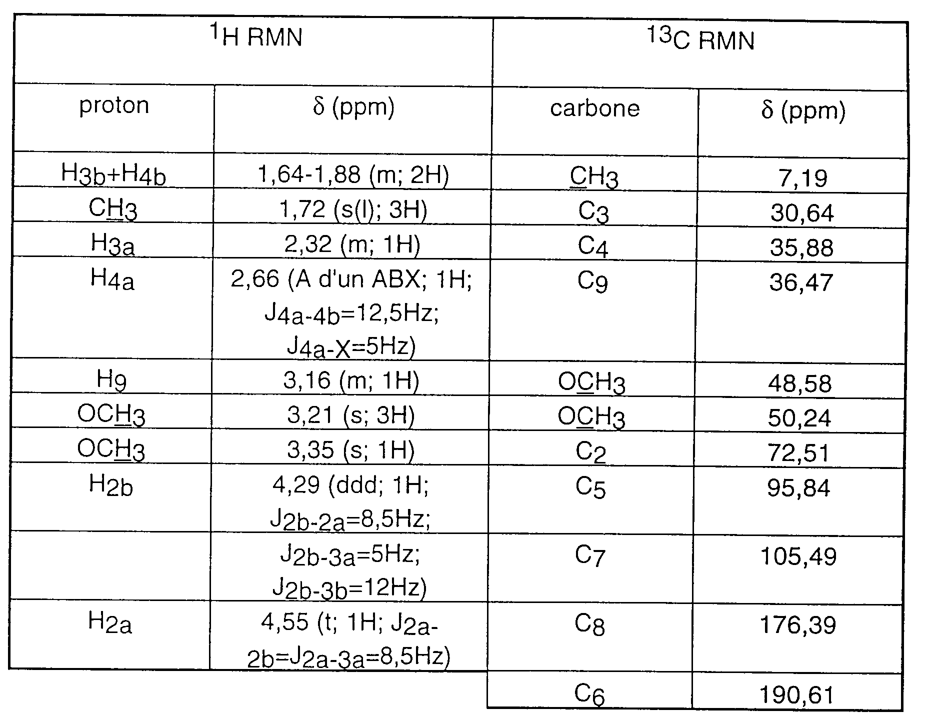
Le spectre IR du composé, effectué dans CCl₄ donne les résultats suivants:

| bandes | cm⁻¹ |
|---|---|
| C-H | 3040-2860 |
| C=O | 1675 |
| C=C | 1650 |

La microanalyse donne les résultats suivants:

| | calculé | trouvé |
|---|---|---|
| % C | 62,25 | 62,40 |
| % H | 7,60 | 7,82 |

Ces analyses correspondent à la structure attendue.
. le 2,3,10,5-tetrahydro-6,6-bis(méthoxy)-7-one-8-méthyl-benzopyranne (n=2, R=CH3)
Pour la chromatographie, l'éluant utilisé est CH₂Cl₂/MeOH 98/2. On obtient, avec un rendement de 83,5%, des cristaux rose pâle.
Les spectres RMN sont les suivants :

| ¹H RMN | | ¹³C RMN | |
|---|---|---|---|
| proton | δ (ppm) | carbone | δ (ppm) |
| H_{4b} | 1,33 (m; 1H) | CH₃ | 7,43 |
| H_{5b} | 1,56 (B d'un ABX; 1H; J_{5b-5a}=13Hz | C₄ | 23,21 |
| | J_{5b-X}=2,5Hz) | C₃ | 26,56 |
| CH₃ | 1,65 (s(I); 3H) | C₁₀ | 31,72 |
| H₃+H₄ₐ | 1,88 (M; 3H) | C₅ | 36,78 |
| H₅ₐ | 2,36 (A d'un ABX; 1H; J_{5a-5b}=12,5Hz; | OCH₃ | 48,58 |
| | J_{5a-X}=4,5Hz) | OCH₃ | 50,47 |
| H₁₀ | 2,74 (m; 1H) | C₂ | 67,87 |
| OCH₃ | 3,10 (s; 3H) | C₆ | 96,14 |
| OCH₃ | 3,28 (s; 1H) | C₈ | 112,34 |
| H_{2b} | 3,96 (m; 1H) | C₉ | 171,80 |
| H₂ₐ | 4,26 (m; 1H) | C₇ | 191,50 |

Le spectre IR dans CCl₄ donne les résultats suivants:

| bandes | cm⁻¹ |
|---|---|
| C-H | 3000-2840 |
| C=O | 1675 |
| C=C | 1630 |

Ces analyses correspondent à la structure attendue.

### Exemple e : synthèse des α-méthoxy-β-hydroxyaromatiques de formule

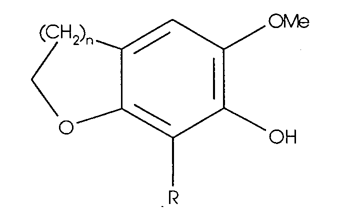

On dissout le composé obtenu à l'exemple d dans l'acide acétique glacial (10 ml/mmol) et l'on chauffe à reflux la solution obtenue pendant 2 heures.
Le solvant est évaporé sous pression réduite et le résidu est chromatographié sur colonne de silice avec l'éluant adéquat.

On synthétise les trois composés suivants:
. le 2,3-dihydro-5-méthoxy-6-hydroxy-benzofuranne (n=1, R=H)
Pour la chromatographie, l'éluant est CH₂Cl₂. On obtient un rendement de 66%.
Les spectres RMN du proton et du carbone 13 donnent les résultats suivants: 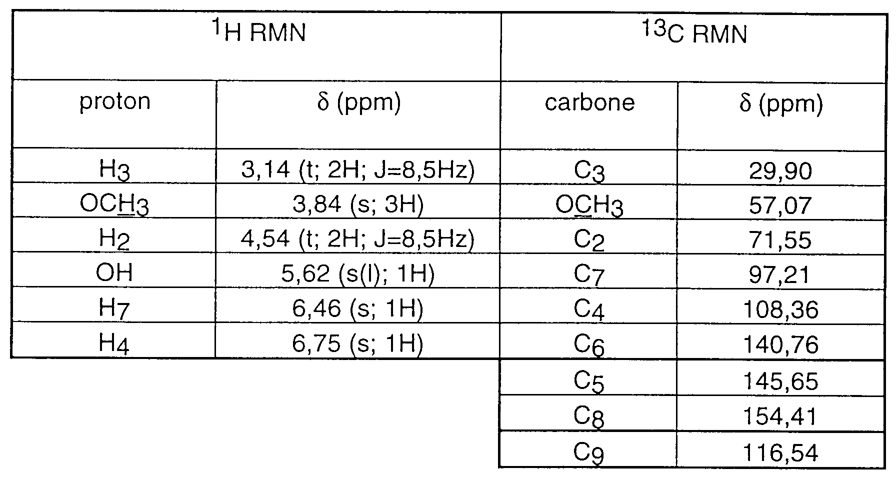
Le spectre IR effectué dans CCl4 donne les résultats suivants:

| bandes | cm⁻¹ |
|---|---|
| OH | 3560 |
| C-H | 3050-2840 |

Ces analyses correspondent à la structure attendue.
. le 2,3-dihydro-5-méthoxy-6-hydroxy-7-méthyl-benzofuranne (n=1, R=CH3)
Pour la chromatographie, l'éluant utilisé est CH₂Cl₂/hexane 50/50. On obtient, avec un rendement de 36%, un produit instable qui devient rouge à l'air.
Le spectre RMN du proton donne les résultats suivants:

| ¹H RMN | |
|---|---|
| proton | δ (ppm) |
| CH₃ | 2,15 (s; 3H) |
| H₃ | 3,15 (t; 2H; J=8,5Hz) |
| OCH₃ | 3,83 (s; 3H) |
| H₂ | 4,53 (t; 2H; J=8,5Hz) |
| OH | 5,70 (s(I); 1H) |
| H₄ | 6,63 (s; 1H) |

Le spectre IR effectué dans CCl₄ donne les résultats suivants:

| bandes | cm⁻¹ |
|---|---|
| OH | 3550 |
| C-H | 3020-2840 |

Ces analyses correspondent à la structure attendue.
. le 2,3-dihydro-6-méthoxy-7-hydroxy-8-méthyl-benzopyranne (n=2, R=CH3)
Pour la chromatographie, l'éluant utilisé est CH₂Cl₂. On obtient, avec un rendement de 36%, une huile jaune pâle.
Les spectres RMN du proton et du carbone 13 donnent les résultats suivants: 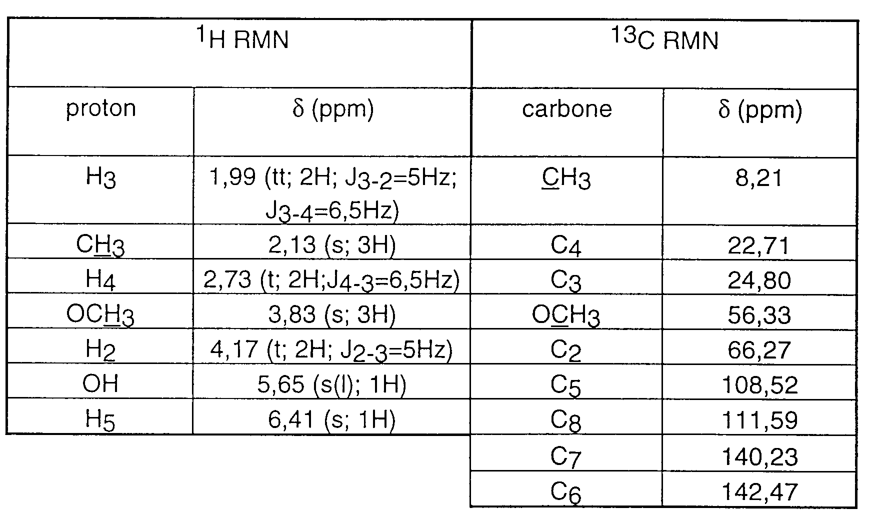
Le spectre IR effectué dans CCl₄ donne les résultats suivants:

| bandes | cm⁻¹ |
|---|---|
| OH | 3560 |
| C-H | 3000-2840 |

Ces analyses correspondent à la structure attendue.

### Exemple 2 : Synthèse de deux composés α-méthoxy-β,δ-diméthylaromatiques de formule

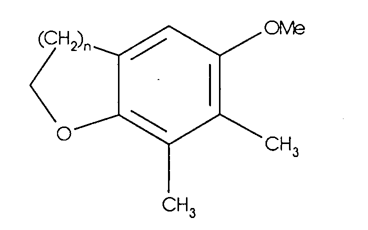

La synthèse des deux composés ci-dessus s'effectue en deux étapes, via la synthèse du composé intermédiaire (exemple f) qui est un α-bis(méthoxy)-β-hydroxy-β-méthyl cyclique.

### Exemple f

Dans un bicol flambé sous argon, on dissout l'acétal cyclique obtenu selon l'exemple d dans du THF anhydre (10 ml/mmol) puis l'on refroidit la solution à -78°C. Du methyl-lithium en solution 1,6M dans l'éther (2 parties pour une partie d'acétal cyclique) est ajouté goutte à goutte. La disparition du produit de départ est suivie par CCM. La réaction est hydrolysée par addition d'une solution saturée en NH₄Cl (10 ml/mmol) et le milieu est extrait au dichlorométhane (3 x 10 ml/mmol). Les phases organiques sont rassemblées, séchées sur sulfate de sodium et concentrées pour conduire à une huile jaune pâle. L'alcool obtenu ne peut être purifié car il est instable et se dégrade sur la silice, même désactivée. Il est donc remis très rapidement en réaction après isolation.

On synthétise de cette manière les deux composés suivants:
. le 2,3,9,4-tetrahydro-5,5-bis(méthoxy)-6-hydroxy-6,7-diméthyl-benzofuranne (n=1)
On obtient un rendement de 93% (reste 7% d'acétal).
Le spectre RMN du proton donne les résultats suivants:

| ¹H RMN | |
|---|---|
| proton | δ (ppm) |
| CH₃ en 6 | 1,29 (s; 3H) |
| H_{3b}+H_{4b} | 1,60 (m; 2H) |
| CH_{3 en 7} | 1,62 (d; 3H; J=2Hz) |
| H₃ₐ | 2,04 (m; 1H) |
| H₄ₐ | 2,24 (A d'un ABX; J_{4a-4b}=13,5Hz; J_{4a-X}=5Hz) |
| H₉ | 2,52 (m; 1H) |
| OH | 2,65 (s(I); 1H) |
| OCH₃ | 3,33 (s; 3H) |
| OCH₃ | 3,44 (s; 1H) |
| H_{2b} | 3,99 (ddd; 1H; J_{2b-2a}=8,5Hz; J_{2b-3a}=5Hz; J_{2b-3b}=11,5Hz) |
| H₂ₐ | 4,22(t; J_{2a-2b}=J₂ₐ₋₃ₐ=8,5Hz) |

Ces analyses correspondent à la structure attendue.
. le 2,3,10,5-tetrahydro-6,6-bis(méthoxy)-7-hydroxy-7,8-diméthyl-benzopyranne (n=2)
On obtient un rendement de 95% (reste 5% d'acétal).
Le spectre RMN du proton donne les résultats suivants:

| ¹H RMN | |
|---|---|
| proton | δ (ppm) |
| H_{4b} | 1,13 (m; 1H) |
| CH₃ en 7 | 1,30 (s; 3H) |
| H_{5b} | 1,43 (B d'un ABX; 1H; J_{5b-5a}=14Hz; J_{5b-X}=3Hz) |
| CH₃ en 8 | 1,55 (s; 3H) |
| H₃ + H₄ₐ | 1,70 (m; 3H) |
| H₅ₐ | 1,96 (A d'un ABX; 1H; J_{5a-5b}=14Hz; J_{5a-X}=5Hz) |
| H₁₀ | 2,16 (m; 1H) |
| OH | 2,73 (s(I); 1H) |
| OCH₃ | 3,28 (s; 3H) |
| OCH₃ | 3,42 (s; 3H) |
| H_{2b} | 4,07 (m; 1H) |
| H₂ₐ | 4,13 (m; 1H) |

Ces analyses correspondent à la structure attendue.

### Exemple g

On dissout l'alcool obtenu selon l'exemple f ci-dessus dans l'acide acétique glacial (10 ml/mmol) et l'on chauffe la solution à 60°C (température du bain d'huile) pendant 1 heure.
Le solvant est évaporé sous pression réduite et le résidu est chromatographié sur colonne de silice avec l'éluant adéquat.

On synthétise de cette manière deux composés:
. le 2,3-dihydro-5-méthoxy-6,7-diméthyl-benzofuranne (n=1)
La chromatographie est effectuée avec pour éluant, un mélange CH₂Cl₂/hexane 50/50. On obtient le composé avec un rendement est 69,5%.
Les spectres RMN du proton et du carbone 13 donnent les résultats suivants: 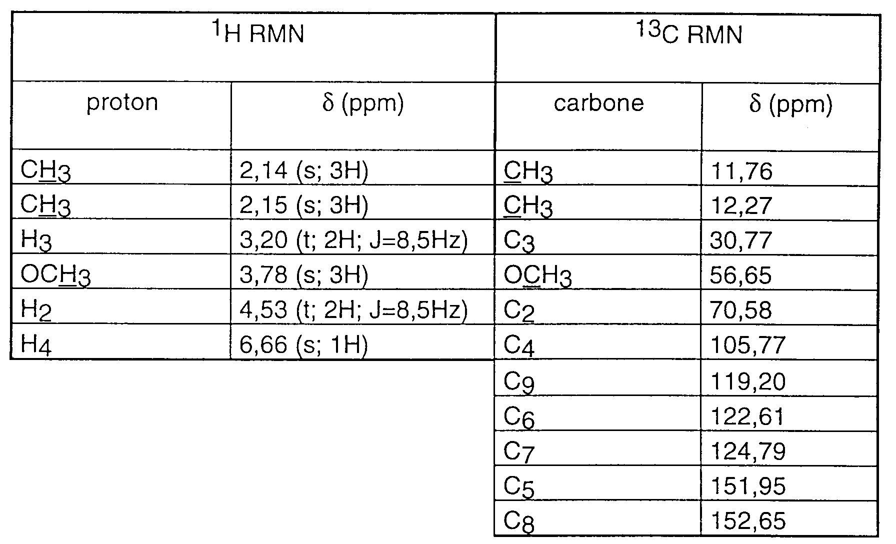
Le spectre IR effectué dans CCl₄ donne les résultats suivants:

| bandes | cm⁻¹ |
|---|---|
| C-H | 3000-2840 |

Ces analyses correspondent à la structure attendue.
. le 2,3-dihydro-6-méthoxy-7,8-diméthyl-benzopyranne (n=2)
La chromatographie est effectuée avec comme éluant un mélange CH₂Cl₂/hexane 50/50. On obtient, avec un rendement de 64%, un mélange 80/20 de composé recherché et de l'alcool correspondant. Il y a déprotection de l'éther lors de la réaction et les deux produits sont inséparables par chromatographie.
Le spectre RMN du proton donne les résultats suivants:

| ¹H RMN | |
|---|---|
| proton | δ (ppm) |
| H₃ | 1,99 (tt; 2H; J₃₋₂=5Hz; J₃₋₄=6,5Hz) |
| CH₃ | 2,14 (s(I); 6H) |
| H₄ | 2,79 (t; 2H; J₄₋₃=6,5Hz) |
| OCH₃ | 3,77 (s; 3H) |
| H₂ | 4,18 (t; 2H; J₂₋₃=5Hz) |
| H₅ | 6,43 (s; 1H) |

Le spectre IR, effectué dans CCl₄, donne les résultats suivants:

| bandes | cm⁻¹ |
|---|---|
| C-H | 3010-2840 |

Ces analyses correspondent à la structure attendue.

### Exemple 3: Synthèse de quatre alkylthio-hydroxyaromatiques de formule

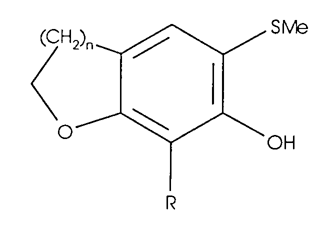

Dans un bicol flambé sous argon et muni d'un condenseur, une suspension de NaH dégraissé dans le dichlorométhane (1 ml/mmol de NaH) est refroidie à 0°C à l'aide d'un bain de glace.
On dissout l'α-cétothioacétal correspondant (1 partie pour 2 parties de NaH) dans le dichlorométhane (1 ml/mmol thioacétal) puis on l'ajoute au goutte à goutte à la canule, à la suspension de NaH.
Quand le dégagement d'hydrogène est terminé, l'α-méthylène lactone (1 partie) dissoute dans le dichlorométhane (1 ml/mmol lactone) est ajoutée sur l'anion du thioacétal goutte à goutte. Après la fin de l'addition, le bain de glace est remplacé par un bain d'huile et le milieu est chauffé à reflux pendant 16 heures. Après refroidissement à température ambiante, la suspension est versée sur une solution aqueuse d'HCl à 10% (10 ml/mmol de thioacétal). La phase organique est décantée et la phase aqueuse est encore extraite 3 fois au dichlorométhane. Les phases organiques rassemblées sont lavées par une solution aqueuse saturée en NaCl, séchées sur sulfate de magnésium puis concentrées.
On dissout le résidu dans du benzène (5 ml/mmol de produit) et une pointe de spatule d'acide p-toluènesulfonique est ajoutée à la solution.
La solution est chauffée à reflux avec un Dean-Stark pendant le temps indiqué ci-après. Après refroidissement, le benzène est évaporé sous pression réduite et le résidu est chromatographié sur colonne de silice avec l'éluant adéquat.

On synthétise de cette manière les quatre composés suivants:
. le 2,3-dihydro-5-méthylthio-6-hydroxy-benzofuranne (n=1, R=H)
Le temps de chauffage est de 120 heures. La chromatographie est effectuée avec comme éluant le CH₂Cl₂. On obtient, avec un rendement de 90%, des cristaux rose pâle dont le point de fusion est de 62-63°C.
Les spectres RMN du proton et du carbone 13 donnent les résultats suivants: 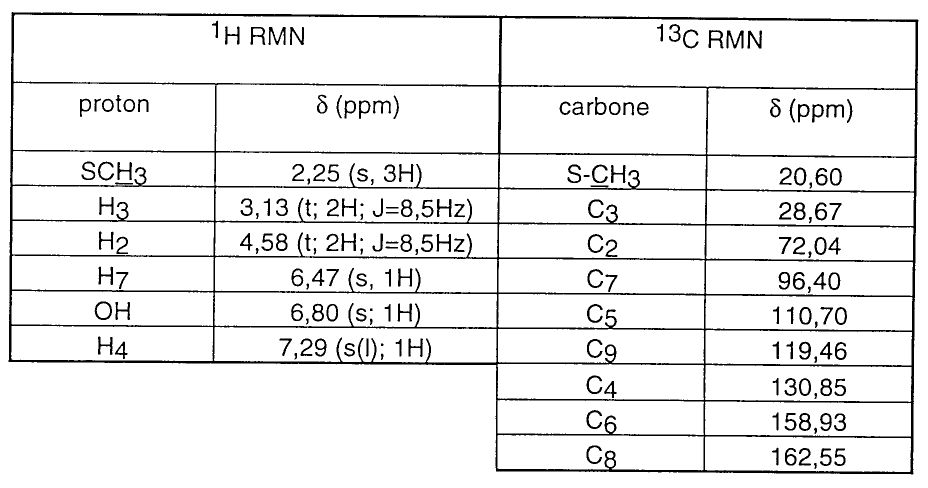
Le spectre IR du composé, effectué dans CCl₄ donne les résultats suivants:

| bandes | cm⁻¹ |
|---|---|
| OH | 3400 |
| C-H | 3000-2860 |

La microanalyse donne les résultats suivants:

| | calculé | trouvé |
|---|---|---|
| % C | 59,48 | 59,32 |
| % H | 5,57 | 5,53 |

Ces analyses correspondent à la structure attendue.
. le 2,3-dihydro-6-méthylthio-7-hydroxy-benzopyranne (n=2, R=H)
Le temps de chauffage est de 72 heures. La chromatographie est effectuée avec comme éluant, le CH₂Cl₂.
Le spectre RMN du proton est le suivant:

| proton | δ (ppm) |
|---|---|
| H₃ | 1,98 (tt; 2H; J₃₋₄=6,5Hz; J₃₋₂=5Hz) |
| SCH₃ | 2,26 (s, 3H) |
| H₄ | 2,71 (t; 2H; J=6,5Hz) |
| H₂ | 4,16 (t; 2H; J=5Hz) |
| H₈ | 6,44 (s, 1H) |
| OH | 6,58 (s; 1H) |
| H₁₀ | 7,17 (s(I); 1H) |

Ces analyses correspondent à la structure attendue.
. le 2,3-dihydro-5-méthylthio-6-hydroxy-7-méthyl-benzofuranne (n=1, R=CH3)
Le temps de chauffage est de 2 heures 30 minutes. La chromatographie est effectuée avec comme éluant, un mélange CH₂Cl₂/hexane 20/80. On obtient une huile incolore avec un rendement de 46%.
Les spectres RMN sont les suivants: 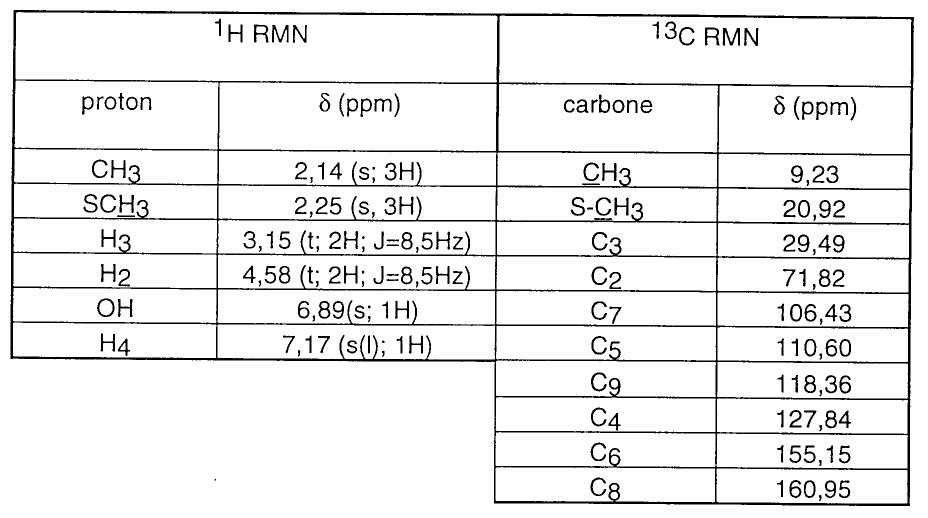
Le spectre IR effectué dans CCl₄ donne les résultats suivants:

| bandes | cm⁻¹ |
|---|---|
| OH | 3400 |
| C-H | 3000-2860 |

Ces analyses correspondent à la structure attendue.
. le 2,3-dihydro-6-méthylthio-7-hydroxy-8-méthyl-benzopyranne (n=2, R=CH3)
Le temps de chauffage est de 4 heures. La chromatographie est effectuée avec comme éluant, le CH₂Cl₂. On obtient avec un rendement de 68%, une huile jaune pâle.
Les spectres RMN sont les suivants: 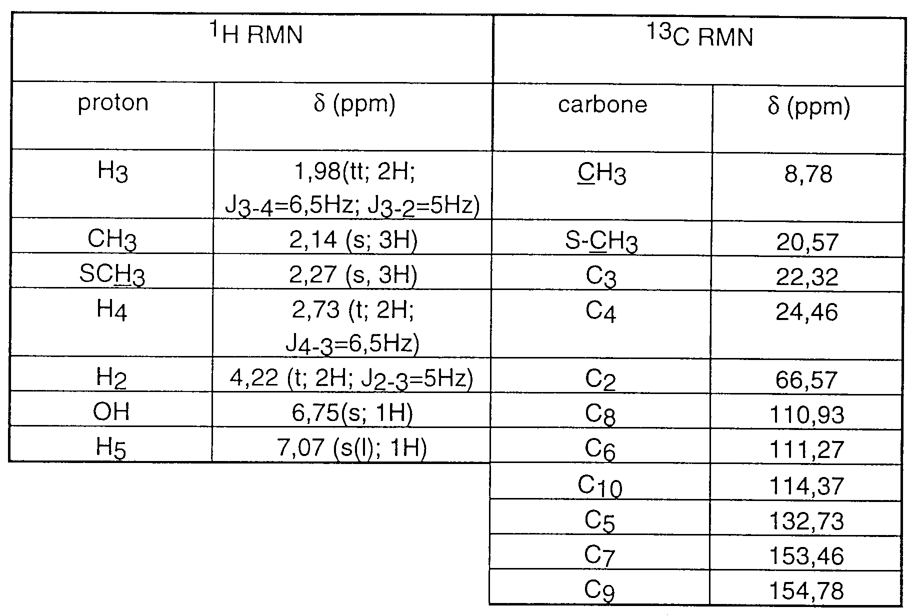
Le spectre IR effectué dans CCl₄ donne les résultats suivants:

| bandes | cm⁻¹ |
|---|---|
| OH | 3400 |
| C-H | 3000-2840 |

Ces analyses correspondent à la structure attendue.

### Exemple 4 : exemple d'utilisation

Les propriétés antioxydantes du 2,3-dihydro-6-hydroxy-5-méthylthiobenzofuranne ont été évaluées par le test du rancimat, sur la vitamine F, en concentration molaire équivalente à 0,5% de B.H.T (butylhydroxytoluène).
Le temps d'induction obtenu est de 108 minutes.
Le produit considéré présente bien une certaine activité antioxydante.

## Revendications

1. Composé chimique de formule (I) 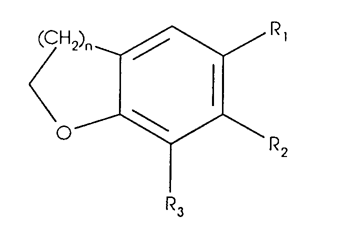 dans laquelle
. n est égal à 1, 2 ou 3,
. R1 est un groupement -SR4 ou -OR4, R4 étant un radical alkyle ayant 1 à 6 atomes de carbone,
. R2 est un groupement -OH, un radical alkyle ayant 1 à 6 atomes de carbone ou un radical alcoxy ayant 1 à 6 atomes de carbone, et
. R3 est un atome d'hydrogène ou un radical alkyle ayant 1 à 6 atomes de carbone,
R1 et R2 ne pouvant être simultanément des groupements -OCH3 lorsque R3 est un atome d'hydrogène et n=2 ou 3.

2. Composé chimique de formule (II) 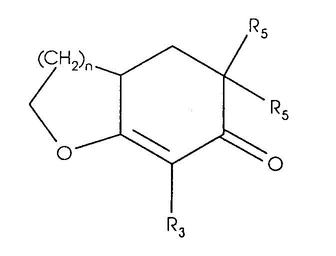 dans laquelle
. n est égal à 1, 2 ou 3,
. R5 est, de manière indépendante, un groupement -SR4 ou -OR4, R4 étant un radical alkyle ayant 1 à 6 atomes de carbone, et
. R3 est un atome d'hydrogène ou un radical alkyle ayant 1 à 6 atomes de carbone.

3. Procédé de préparation des composés de formule (II) dans laquelle chaque R5 est un groupement -SR4, R4 étant un radical alkyle ayant 1 à 6 atomes de carbone, dans lequel on fait réagir, selon la réaction d'annélation 3C+3C de Michael Claisen, une α-méthylènelactone adéquate avec l'anion d'un α-cétothioacétal adéquat, de manière à obtenir un mélange céto-énolique, puis l'on déshydrate partiellement ledit mélange de manière à obtenir le dialkylthioacétal de formule (II) recherché.

4. Procédé de préparation des composés de formule (II) dans laquelle chaque R5 est un groupement -OR4, R4 étant un radical alkyle ayant 1 à 6 atomes de carbone, dans lequel on fait réagir, selon la réaction d'annélation 3C+3C de Michael Claisen, une α-méthylène-lactone adéquate avec l'anion d'un α-cétothioacétal adéquat, de manière à obtenir un mélange céto-énolique, puis l'on déshydrate partiellement ledit mélange de manière à obtenir un dialkylthioacétal que l'on transforme, en présence d'acétate de mercure, en dialcoxyacétal de formule (II).

5. Procédé de préparation des composés de formule (I) dans laquelle R1 est un radical alcoxy ayant 1 à 6 atomes de carbone et R2 est un groupement -OH ou un radical alcoxy ayant 1 à 6 atomes de carbone, dans lequel on fait réagir, selon la réaction d'annélation 3C+3C de Michael Claisen, une α-méthylènelactone adéquate avec l'anion d'un α-cétothioacétal adéquat, de manière à obtenir un mélange céto-énolique, puis l'on déshydrate partiellement ledit mélange de manière à obtenir un dialkylthioacétal que l'on transforme, en présence d'acétate de mercure, en dialcoxyacétal, puis l'on hydrolyse ledit dialcoxyacétal de manière à obtenir les composés de formule (I) recherchés.

6. Procédé de préparation des composés de formule (I) dans laquelle R1 est un radical alcoxy ayant 1 à 6 atomes de carbone, et R2 est un radical alkyle ayant 1 à 6 atomes de carbone, dans lequel on fait réagir, selon la réaction d'annélation 3C+3C de Michael Claisen, une α-méthylènelactone adéquate avec l'anion d'un α-cétothioacétal adéquat, de manière à obtenir un mélange céto-énolique, puis l'on déshydrate partiellement ledit mélange de manière à obtenir un dialkylthioacétal que l'on transforme en dialcoxyacétal, puis l'on ajoute audit dialcoxyacétal du méthyl-lithium et on l'hydrolyse de manière à obtenir les composés de formule (I) recherchés.

7. Procédé de préparation des composés de formule (I) dans laquelle R1 est un groupement -SR4, R4 étant un radical alkyle ayant 1 à 6 atomes de carbone, dans lequel on fait réagir, selon la réaction d'annélation 3C+3C de Michael Claisen, une α-méthylènelactone adéquate avec l'anion d'un α-cétothioacétal adéquat, de manière à obtenir un mélange céto-énolique que l'on déshydrate et désulfure partiellement dans le benzène, en présence d'acide p-toluène sulfonique, de manière à obtenir les composés de formule (I) recherchés.

8. Utilisation des composés de formule (II) pour la préparation des composés de formule (I).

9. Utilisation d'un composé de formule (I) comme agent antioxydant.

10. Utilisation selon la revendication 9, dans une composition cosmétique ou pharmaceutique.

11. Composition cosmétique ou pharmaceutique comprenant, dans un véhicule approprié, au moins un composé de formule (I).

12. Composition cosmétique ou pharmaceutique comprenant, dans un véhicule approprié, au moins un composé de formule (I) en tant qu'agent antioxydant.

## Claims

1. Chemical compound of formula (I) 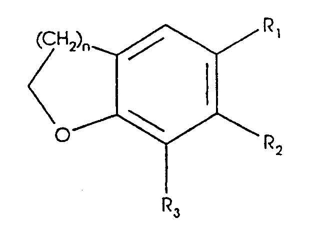 in which
• n is equal to 1, 2 or 3,
• R₁ is a group -SR₄ or -OR₄, R₄ being an alkyl radical having 1 to 6 carbon atoms,
• R₂ is an -OH group, an alkyl radical having 1 to 6 carbon atoms or an alkoxy radical having 1 to 6 carbon atoms, and
• R₃ is a hydrogen atom or an alkyl radical having 1 to 6 carbon atoms,
it not being possible for R₁ and R₂ simultaneously to be -OCH₃ groups when R₃ is a hydrogen atom and n = 2 or 3.

2. Chemical compound of formula (II) 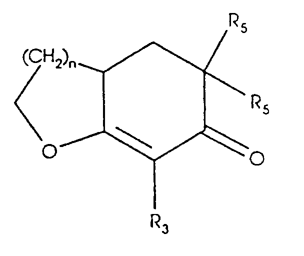 in which
• n is equal to 1, 2 or 3,
• R₅ is, independently, a group -SR₄ or -OR₄, R₄ being an alkyl radical having 1 to 6 carbon atoms, and
• R₃ is a hydrogen atom or an alkyl radical having 1 to 6 carbon atoms.

3. Process for the preparation of the compounds of formula (II) in which each R₅ is a group -SR₄, R₄ being an alkyl radical having 1 to 6 carbon atoms, in which a suitable α-methylenelactone is reacted, according to the 3C+3C Michael Claisen annelation reaction, with the anion of a suitable α-keto thioacetal, so as to obtain a keto-enol mixture, followed by partial dehydration of the said mixture so as to obtain the desired dialkylthioacetal of formula (II).

4. Process for the preparation of the compounds of formula (II) in which each R₅ is a group -OR₄, R₄ being an alkyl radical having 1 to 6 carbon atoms, in which a suitable α-methylenelactone is reacted, according to the 3C+3C Michael Claisen annelation reaction, with the anion of a suitable α-keto thioacetal, so as to obtain a keto-enol mixture, followed by partial dehydration of the said mixture so as to obtain a dialkylthioacetal which is converted, in the presence of mercury acetate, into a dialkoxyacetal of formula (II).

5. Process for the preparation of the compounds of formula (I) in which R₁ is an alkoxy radical having 1 to 6 carbon atoms and R₂ is an -OH group or an alkoxy radical having 1 to 6 carbon atoms, in which a suitable α-methylenelactone is reacted, according to the 3C+3C Michael Claisen annelation reaction, with the anion of a suitable α-keto thioacetal, so as to obtain a keto-enol mixture, followed by partial dehydration of the said mixture so as to obtain a dialkylthioacetal which is converted, in the presence of mercury acetate, into a dialkoxyacetal, and then hydrolysis of the said dialkoxyacetal so as to obtain the desired compounds of formula (I).

6. Process for the preparation of the compounds of formula (I) in which R₁ is an alkoxy radical having 1 to 6 carbon atoms and R₂ is an alkyl radical having 1 to 6 carbon atoms, in which a suitable α-methylenelactone is reacted, according to the 3C+3C Michael Claisen annelation reaction, with the anion of a suitable α-keto thioacetal, so as to obtain a keto-enol mixture, followed by partial dehydration of the said mixture so as to obtain a dialkylthioacetal which is converted into a dialkoxyacetal, and methyllithium is then added to the said dialkoxyacetal and it is hydrolysed so as to obtain the desired compounds of formula (I).

7. Process for the preparation of the compounds of formula (I) in which R₁ is a group -SR₄, R₄ being an alkyl radical having 1 to 6 carbon atoms, in which a suitable α-methylenelactone is reacted, according to the 3C+3C Michael Claisen annelation reaction, with the anion of a suitable α-keto thioacetal, so as to obtain a keto-enol mixture which is partially dehydrated and desulphurized in benzene, in the presence of p-toluenesulphonic acid, so as to obtain the desired compounds of formula (I).

8. Use of the compounds of formula (II) for the preparation of the compounds of formula (I).

9. Use of a compound of formula (I) as an antioxidant.

10. Use according to Claim 9, in a cosmetic or pharmaceutical composition.

11. Cosmetic or pharmaceutical composition comprising at least one compound of formula (I) in a suitable carrier.

12. Cosmetic or pharmaceutical composition comprising at least one compound of formula (I) as antioxidant, in a suitable carrier.

## Patentansprüche

1. Chemische Verbindung der Formel (I) 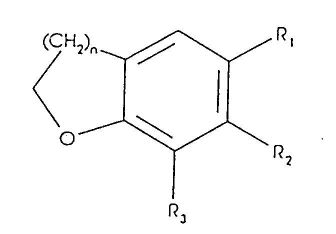 in der
n den Wert 1, 2 oder 3 hat,
R₁ eine -SR₄ oder -OR₄-Gruppe bedeutet, wobei R₄ einen Alkylrest mit 1 bis 6 Kohlenstoffatomen darstellt,
R₂ eine -OH-Gruppe, einen Alkylrest mit 1 bis 6 Kohlenstoffatomen oder einen Alkoxyrest mit 1 bis 6 Kohlenstoffatomen bedeutet und
R₃ ein Wasserstoffatom oder einen Alkylrest mit 1 bis 6 Kohlenstoffatomen bedeutet,
wobei R₁ und R₂ nicht gleichzeitig -OCH₃-Gruppen bedeuten können, wenn R₃ ein Wasserstoffatom ist und n = 2 oder 3.

2. Chemische Verbindung der Formel (II) 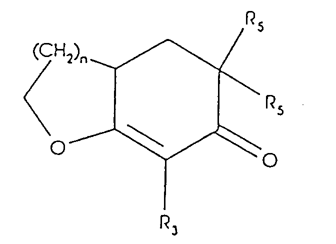 in der n den Wert 1, 2 oder 3 hat, die Reste R₅ unabhängig voneinander eine -SR₄- oder -OR₄-Gruppe bedeuten, wobei R₄ einen Alkylrest mit 1 bis 6 Kohlenstoffatomen darstellt, und R₃ ein Wasserstoffatom oder einen Alkylrest mit 1 bis 6 Kohlenstoffatomen bedeutet.

3. Verfahren zur Herstellung der Verbindungen der Formel (II), in der die Reste R₅ jeweils eine -SR₄-Gruppe bedeuten, wobei R₄ einen Alkylrest mit 1 bis 6 Kohlenstoffatomen darstellt, wobei man in einer 3C+3C-Anellierung gemäß Michael-Claisen ein entsprechendes α-Methylenlacton mit dem Anion eines entsprechenden α-Ketothioacetals unter Bildung eines Keto-Enol-Gemisches umsetzt und anschließend dieses Gemisch partiell dehydratisiert, wodurch man das gewünschte Dialkylthioacetal der Formel (II) erhält.

4. Verfahren zur Herstellung der Verbindungen der Formel (II), in der die einzelnen Reste R₅ jeweils eine -OR₄-Gruppe bedeuten, wobei R₄ einen Alkylrest mit 1 bis 6 Kohlenstoffatomen darstellt, wobei man durch eine 3C+3C-Anellierung gemäß Michael-Claisen ein entsprechendes α-Methylenlacton mit dem Anion eines entsprechenden α-Ketothioacetals unter Bildung eines Keto-Enol-Gemisches umsetzt und anschließend dieses Gemisch unter Bildung eines Dialkylthioacetals partiell dehydratisiert, wobei man diese Verbindung dann in Gegenwart von Quecksilberacetat in das Dialkoxyacetal der Formel (II) umwandelt.

5. Verfahren zur Herstellung der Verbindungen der Formel (I), in der R₁ einen Alkoxyrest mit 1 bis 6 Kohlenstoffatomen bedeutet und R₂ eine -OH-Gruppe oder einen Alkoxyrest mit 1 bis 6 Kohlenstoffatomen bedeutet, wobei man durch eine 3C+3C-Anellierung gemäß Michael-Claisen ein entsprechendes α-Methylenlacton mit dem Anion eines entsprechenden α-Ketothioacetals unter Bildung eines Keto-Enol-Gemisches umsetzt, anschließend dieses Gemisch unter Bildung eines Dialkylthioketals partiell dehydratisiert, das gebildete Produkt in Gegenwart von Quecksilberacetat in ein Dialkoxyacetal umwandelt und anschließend das Dialkoxyacetal unter Bildung der gewünschten Verbindung der Formel (I) hydrolysiert.

6. Verfahren zur Herstellung der Verbindungen der Formel (I), in der R₁ einen Alkoxyrest mit 1 bis 6 Kohlenstoffatomen bedeutet und R₂ einen Alkylrest mit 1 bis 6 Kohlenstoffatomen bedeutet, wobei man durch eine 3C+3C-Anellierung gemäß Michael-Claisen ein entsprechendes α-Methylenlacton mit dem Anion eines entsprechenden α-Ketothioacetals unter Bildung eines Keto-Enol-Gemisches umsetzt, anschließend dieses Gemisch unter Bildung eines α-Dialkylthioacetals partiell dehydratisiert, das Produkt in ein Dialkoxyacetal umwandelt, sodann das Dialkoxyacetal mit Methyllithium versetzt und unter Bildung der gewünschten Verbindung der Formel (I) hydrolysiert.

7. Verfahren zur Herstellung der Verbindungen der Formel (I), in der R₁ eine -SR₄-Gruppe bedeutet, worin R₄ einen Alkylrest mit 1 bis 6 Kohlenstoffatomen bedeutet, wobei man durch eine 3C+3C-Anellierung gemäß Michael-Claisen ein entsprechendes α-Methylenlacton mit dem Anion eines entsprechenden α-Ketothioacetals unter Bildung eines Keto-Enol-Gemisches umsetzt und dieses Gemisch in Benzol in Gegenwart von p-Toluolsulfonsäure partiell dehydratisiert und desulfuriert, wodurch man die gewünschte Verbindung der Formel (I) erhält.

8. Verwendung der Verbindungen der Formel (II) zur Herstellung der Verbindungen der Formel (I).

9. Verwendung der Verbindungen der Formel (I) als Antioxidationsmittel.

10. Verwendung nach Anspruch 9 in einer kosmetischen oder pharmazeutischen Zusammensetzung.

11. Kosmetische oder pharmazeutische Zusammensetzung, enthaltend mindestens eine Verbindung der Formel (I) in einem geeigneten Träger.

12. Kosmetische oder pharmazeutische Zusammensetzung, enthaltend mindestens eine Verbindung der Formel (I) als Antioxidationsmittel in einem geeigneten Träger.
